(19) Europäisches Patentamt
European Patent Office
Office européen des brevets

(11) Veröffentlichungsnummer: **0 534 501 A2**

(12) # EUROPÄISCHE PATENTANMELDUNG

(21) Anmeldenummer: **92120256.0**

(22) Anmeldetag: **28.06.90**

(51) Int. Cl.[5]: **C07C 279/04**, A01N 47/44, C07D 295/215, C07D 279/10, C07D 279/12, C07C 279/08, C07D 211/16, C07D 265/30

Diese Anmeldung is am 27 - 11 - 1992 als Teilanmeldung zu der unter INID-Kode 60 erwähnten Anmeldung eingereicht worden.

(30) Priorität: **06.07.89 DE 3922232**

(43) Veröffentlichungstag der Anmeldung: **31.03.93 Patentblatt 93/13**

(60) Veröffentlichungsnummer der früheren Anmeldung nach Art. 76 EPÜ: **0 406 699**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Zipplies, Matthias, Dr.**
**Kastanienweg 1**
**W-6945 Hirschberg(DE)**
Erfinder: **Sauter, Hubert, Dr.**
**Neckarpromenade 20**
**W-6800 Mannheim 1(DE)**
Erfinder: **Roehl, Franz, Dr.**
**Karl-Otto-Braun-Strasse 8**
**W-6700 Ludwigshafen(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

(54) **Fungizide Guanidine.**

(57) Guanidine I

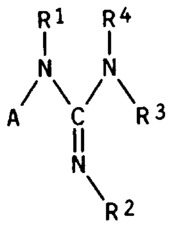

(I)

(A = in para-Position substituiertes Benzyl; $R^1$, $R^2$, $R^3$ = H, $C_1$-$C_4$-Alkyl; $R^4$ = $C_5$-$C_{18}$-Alkyl, das durch O unterbrochen sein kann, $C_5$-$C_{18}$-Alkenyl, $C_4$-$C_{18}$-Alkinyl oder Phenyl-$C_1$-$C_6$-alkyl, wobei diese Gruppen bis zu drei weitere Substituenten tragen können und der Phenylteil des Phenylalkyls zusätzlich 1 Phenoxygruppe oder bis zu 3 $C_2$-$C_4$-Alkenylgruppen, $C_1$-$C_4$-Alkoxy-$C_1$-$C_4$-alkylgruppen, $C_1$-$C_6$-Alkyl- oder Halogenalkylgruppen tragen kann, $C_5$-$C_6$-Cycloalkyl-$C_1$-$C_8$-alkyl, wobei der Cyclus bis zu drei Substituenten tragen kann; und $C_3$-$C_4$-Alkyl, das durch O unterbrochen sein kann oder $C_4$-Alkenyl, die beide bis zu drei weitere Substituenten tragen können; $R^3$ + $R^4$ = $C_5$-$C_6$-Heterocyclus, der bis zu 3 weitere Substituenten tragen und durch O unterbrochen sein kann)

EP 0 534 501 A2

sowie die Salze I•HX und die Metallkomplexe von I.

Die Verbindungen I eignen sich als Fungizide.

Die vorliegende Erfindung betrifft neue Guanidine der Formel I

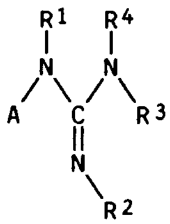

(I)

in der die Substituenten die folgende Bedeutung haben:

A eine Benzylgruppe, die in para-Position durch $C_1$-$C_{10}$-Alkyl oder $C_1$-$C_{10}$-Alkoxy substituiert ist, wobei der Substituent noch eine Hydroxyl- oder $C_1$-$C_6$-Alkoxygruppe tragen kann;

$R^1$, $R^2$, $R^3$ Wasserstoff oder $C_1$-$C_4$-Alkylgruppen;

$R^4$ eine $C_5$-$C_{18}$-Alkylgruppe, die durch Sauerstoff unterbrochen sein kann, eine $C_5$-$C_{18}$-Alkenylgruppe, eine $C_4$-$C_{18}$-Alkinylgruppe oder eine Phenyl-$C_1$-$C_6$-alkylgruppe, wobei diese Gruppen bis zu drei der folgenden Substituenten tragen können: Hydroxyl, Halogen, Cyan, $C_1$-$C_7$-Alkoxy oder bis zu zwei Amino-, $C_1$-$C_4$-Alkylamino- oder Di-($C_1$-$C_4$)-alkylamino-Substituenten und wobei der Phenylteil der Phenylalkylgruppe zusätzlich eine Phenoxygruppe oder bis zu 3 $C_2$-$C_4$-Alkenylgruppen, $C_1$-$C_4$-Alkoxy-$C_1$-$C_4$-alkylgruppen oder $C_1$-$C_6$-Alkylgruppen, die unsubstituiert oder partiell oder vollständig halogeniert sein können, tragen kann, oder eine $C_5$-$C_6$-Cycloalkyl-$C_1$-$C_8$-alkylgruppe, wobei der Cycloalkylring bis zu drei $C_1$-$C_4$-Alkylgruppen oder bis zu zwei Hydroxyl- oder Trifluormethylgruppen tragen kann; und

eine $C_3$-$C_4$-Alkylgruppe, die durch Sauerstoff unterbrochen sein kann oder eine $C_4$-Alkenylgruppe, wobei diese Gruppen bis zu 3 der folgenden Substituenten tragen können: Hydroxyl, Halogen, Cyan, $C_1$-$C_7$-Alkoxy oder bis zu zwei Amino-, $C_1$-$C_4$-Alkylamino- oder $C_2$-$C_8$-Dialkylaminogruppen; oder gemeinsam mit dem Rest $R^3$ und dem Stickstoffatom einen 5- oder 6-gliedrigen heterocyclischen Ring, der mit $C_1$-$C_6$-Alkyl, Phenyl, $C_1$-$C_6$-Alkylphenyl ein- bis dreifach substituiert und durch ein Sauerstoffatom unterbrochen sein kann;

sowie die pflanzenverträglichen mineralsauren Salze I•HX und Metallkomplexe von I.

Außerdem betrifft die Erfindung Verfahren zur Herstellung dieser Verbindungen, ihre Verwendung als Fungizide und fungizide Mittel, welche diese Verbindungen als wirksame Substanzen enthalten.

Aus der Monographie "Chemie der Pflanzenschutz- und Schädlingsbekämpfungsmittel", Band 4, Springer Verlag 1977, S. 145ff., sind fungizide Alkylguanidin-Salze (Dodine) und Bis-Alkylguanidin-amin-Salze (Guazatine) bekannt, die an einem der Guanidin-Stickstoffatome eine Alkylgruppe tragen bzw. deren Guanidinogruppen durch eine Azaalkylengruppe miteinander verknüpft sind.

Guanidin-Derivate mit fungizider Wirkung, die an einem Stickstoffatom mit einer Arylgruppe und an den beiden anderen Stickstoffatomen mit Alkyl- und Cycloalkylgruppen substituiert sind, sind in der DE-A1-31 08 564 beschrieben.

Die Wirkungen dieser Verbindungen können jedoch, besonders bei kleinen Aufwandmengen und -konzentrationen, nur bedingt befriedigen. Insbesondere führen sie teilweise zu Schäden an den Nutzpflanzen, so daß der Erfindung als Aufgabe zugrunde lag, neue fungizid wirksame Verbindungen zu finden, die auch in kleineren Aufwandmengen bessere fungizide Eigenschaften als die bisher bekannten Verbindungen aufweisen, ohne dabei die Nutzpflanzen nennenswert zu schädigen.

Demgemäß wurden die eingangs definierten Guanidine der Formel I gefunden. Weiterhin wurden Verfahren zur Herstellung dieser Guanidine gefunden.

Wenn $R^1$ und/oder $R^3$ Wasserstoff bedeuten, können die Verbindungen I auch in tautomeren Formen vorliegen, die von der Formel I umfaßt werden.

Im einzelnen haben die Substituenten in den erfindungsgemäßen Verbindungen I die folgende Bedeutung:

A

- Benzyl, wobei diese Gruppe in para-Position einen der folgenden Substituenten tragen kann:

- eine verzweigte oder unverzweigte $C_1$-$C_{10}$-Alkylgruppe, insbesondere eine $C_1$-$C_6$-Alkylgruppe wie die Methyl-, Ethyl-, Propyl-, Isopropyl-, Butyl-, Isobutyl-, sek.-Butyl-, tert.-Butyl-, 1,1-Dimethylethyl, 1,1-Dimethylpropyl-, 2,3-Dimethylpropyl-, 1,1,2-Trimethylpropyl-, 2-Hydroxy-prop-2-ylgruppe oder eine 2-Methoxy-prop-2-ylgruppe;
- eine $C_1$-$C_{10}$-Alkoxygruppe, insbesondere eine $C_1$-$C_6$-Alkoxygruppe wie die Methoxy-, Ethoxy-, Isopropoxy-, n-Butoxy-, tert.-Butoxygruppe oder die Hexyloxygruppe;

besonders bevorzugt unter den Resten A werden Benzyl, p-Methylbenzyl, p-Ethylbenzyl, p-Isopropylbenzyl, p-tert.-Butylbenzyl, p-(2,3-Dimethylpropyl)-benzyl, p-(1,1-Dimethylethyl)-benzyl, p-(1,1,2-Trimethylpropyl)-benzyl, p-(2-Hydroxy-prop-2-yl)-benzyl, p-(2-Methoxy-prop-2-yl)-benzyl, p-Methoxybenzyl oder p-tert.-Butoxybenzyl;

$R^1$, $R^2$, $R^3$ vorzugsweise Wasserstoff, sowie Methyl, Ethyl, Propyl, Isopropyl, n-Butyl oder Isobutyl;

$R^4$

- eine verzweigte oder unverzweigte $C_5$-$C_{18}$-Alkylgruppe, die durch Sauerstoff unterbrochen sein kann, bevorzugt 2,2-Dimethylpropyl, 3-Methylbutyl, 3,3-Dimethylbutyl, n-Pentyl, 4,4-Dimethylpentyl, n-Hexyl, 2-Ethylhexyl, 3,5,5-Trimethylhexyl, n-Heptyl, n-Octyl, 5-Methyloct-2-yl, 2-Hydroxyoctyl, 8-Hydroxyoctyl, 8-Fluoroctyl, 8-Chloroctyl, 2,5,7,7-Tetramethyloctyl, n-Nonyl, n-Decyl, n-Dodecyl, n-Tridecyl, iso-Tridecyl, 6,10-Dimethylundec-2-yl, 6,10,14-Trimethylpentadec-2-yl, n-Hexadecyl, n-Octadecyl, 4-(4-tert.-Butoxy)but-2-yl, tert.-Butoxy-pentyl, 6-Ethyl-4-oxa-decyl, 3-Diethylaminopropyl;
- eine verzweigte oder unverzweigte $C_5$-$C_{18}$-Alkenyl- oder $C_4$-$C_{18}$-Alkinylgruppe, insbesondere die Dimethylallyl- oder But-2-inylgruppe;
- eine Phenyl-$C_1$-$C_6$-alkylgruppe, insbesondere eine Phenyl-$C_1$-$C_4$-alkylgruppe, die eine Phenoxygruppe oder bis zu drei der folgenden Reste tragen kann:
  - Hydroxyl;
  - Halogen, darunter vor allem Fluor oder Chlor;
  - Cyan;
  - verzweigtes oder unverzweigtes $C_1$-$C_7$-Alkoxy, insbesondere $C_1$-$C_4$-Alkoxy wie die Methoxy, Ethoxy, Isopropoxy, n-Butoxy oder tert.-Butoxy oder
  - bis zu 2 Amino-, $C_1$-$C_4$-Alkylamino- und/oder Di-($C_1$-$C_4$)-alkylamino-Substituenten, insbesondere Amino, Dimethylamino und Diethylamino;
  - eine Phenoxygruppe am Phenylteil der Phenylalkylgruppe;
  - bis zu drei der folgenden Substituenten am Phenylteil der Phenylalkylgruppe:
    - verzweigtes oder unverzweigtes $C_1$-$C_4$-Alkoxy-$C_1$-$C_4$-alkyl, insbesondere $C_1$-$C_4$-Alkoxy-$C_1$-$C_2$-alkyl wie Methoxymethyl, Ethoxymethyl oder Ethoxyethyl;
    - verzweigtes oder unverzweigtes $C_2$-$C_4$-Alkenyl, insbesondere die Ethenyl- oder Isopropenylgruppe,
    - $C_1$-$C_4$-Alkoxy-$C_1$-$C_4$-alkyl wie Methoxymethyl
    - verzweigtes oder unverzweigtes $C_1$-$C_6$-Alkyl oder partiell oder vollständig halogeniertes $C_1$-$C_6$-Halogenalkyl, insbesondere Methyl, Trichlormethyl, Trifluormethyl, Ethyl, Propyl, Isopropyl, n-Butyl, tert.-Butyl, 1,1-Dimethylpropyl, 1,1,2-Trimethylpropyl, 2,4,4-Trimethylpentyl oder Perfluorpentyl;

  besonders bevorzugt werden die Reste Benzyl, 4-Hydroxybenzyl, 3-Chlorbenzyl, 4-Chlorbenzyl, 2,4-Dichlorbenzyl, 4-Cyanobenzyl, 4-Methylbenzyl, 4-Trifluormethylbenzyl, 4-Trichlormethylbenzyl, 4-Isopropylbenzyl, 4-Isopropenylbenzyl, 3-tert.-Butylbenzyl, 4-tert.-Butylbenzyl, 4-(1,1-Dimethylpropyl)benzyl, 4-(1,1,2-Trimethylpropyl)benzyl, 4-(2,4,4-Trimethylpentyl)benzyl, 4-(1-Hydroxy-1-methylethyl)-benzyl, 3,5-Di-tert.-butyl-4-hydroxybenzyl, 4-Methoxybenzyl, 4-(1-Methoxy-1-methylethyl)benzyl, 4-n-Butoxybenzyl oder 4-tert.-Butoxybenzyl, 1-Phenylethyl, Phenethyl, 4-Methoxyphenethyl, 4-tert.-Butylphenethyl, 3-Phenylpropyl, 3-(4-tert.-Butylphenyl)-2-methylpropyl oder 4-Phenyl-but-2-yl;
  - eine $C_5$-$C_6$-Cycloalkyl-$C_1$-$C_8$-alkylgruppe, wobei der Cycloalkylring bis zu drei $C_1$-$C_4$-Alkylgruppen wie Methyl und tert.-Butyl oder bis zu zwei Hydroxyl- oder Trifluormethylgruppen tragen kann, insbesondere eine $C_5$-$C_6$-Cycloalkyl-$C_1$-$C_4$-alkylgruppe wie Cyclohexylmethyl, 4-tert.-Butylcyclohexylmethyl, 4-Trifluormethylcyclohexylmethyl, 4-Hydroxycyclohexylmethyl, 4-tert.-Butoxycyclohexylmethyl, Cyclohexylethyl, Cyclohexylpropyl oder 3-(4-tert.-Butylcyclohexyl)-2-methylpropyl;

und

- eine verzweigte oder unverzweigte C3-$C_4$-Alkylgruppe, die durch Sauerstoff unterbrochen sein kann, insbesondere die Isopropyl-, tert.-Butyl- oder die 2-Hydroxypropylgruppe;
- eine $C_4$-Alkylengruppe, insbesondere die But-2-enylgruppe;

oder gemeinsam mit dem Rest $R^3$ und dem Stickstoffatom ein 5- oder 6-gliedriger Heterocyclus, der mit $C_1$-$C_6$-Alkyl, Phenyl, $C_1$-$C_6$-Alkylphenyl ein- bis dreifach substituiert und durch ein Sauerstoffatom unterbrochen sein kann, insbesondere eine 2-(1,5-Dimethylhexyl)-pyrrolidinyl-,2-(2,4,4-Trimethylpentyl)-pyrrolidinyl-, 3-Phenylpyrrolidinyl-, 3-(4-tert.-Butylphenyl)-pyrrolidinyl, 3-(4-tert.-Butylphenyl)-4-methyl-pyrrolidinyl-, Piperidinyl-, 4-tert.-Butylpiperidinyl-, 4-(4-tert.-Butylphenyl)-piperidinyl-, Morpholinyl- oder 2,6-Dimethyl-morpholin-4-yl-gruppe.

Besonders geeignete Verbindungen I sind der Tabelle zu entnehmen, wobei insbesondere diejenigen bevorzugt werden, die folgende Substituenten tragen:

A

- p-tert.-Butylbenzyl;

$R^1$

- Wasserstoff;

$R^2$

- Wasserstoff oder $C_1$-$C_4$-Alkyl;

$R^3$

- Wasserstoff oder Methyl;

$R^4$

- $C_6$-$C_{14}$-Alkyl, insbesondere 2-Ethylhexyl und 6,10-Dimethylundec-2-yl;
- p-($C_1$-$C_6$-Akyl)-phenyl-$C_1$-$C_4$-alkyl, insbesondere p-tert.-Butylbenzyl, 3-(p-tert.-Butylphenyl)-2-methyl-propyl und p-(2,3-Dimethylbut-2-yl)-benzyl;
- gemeinsam mit dem Rest $R^3$ und dem Stickstoffatom 4-tert.-Butylphenyl-pyrrolidinyl.

Als Säureadditionssalze eignen sich die pflanzenverträglichen Salze von solchen Säuren, welche die fungizide Wirkung von I nicht beeinträchtigen, also z.B. die Iodide, Chloride, Bromide, Hydrochloride und -bromide, Sulfate, Dodecylsulfate, Nitrate, Carbonate, Phosphate, Formiate, Acetate, Propiate, Benzoate, Oxalate, Naphthalinsulfonate, Dodecylbenzolsulfonate, Lactate und die Salze mit dem Anion des Saccharins. Bevorzugt werden die Jodide, Chloride und Acetate.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und mineralsauren Salzen, beispielsweise den Chloriden oder Sulfaten, der Metalle her.

Ganz besonders gut geeignet sind die folgenden Verbindungen I:

N-1-(p-tert.-Butylbenzyl)-N-2-(2-ethylhexyl)-guanidin-hydrojodid,

N-1-(p-tert.-Butylbenzyl)-N-2-(p-tert.-butylbenzyl)-N-3-methyl-guanidin-hydrojodid,

1-([(3,3-Dimethylcyclohexyl)-amino]-iminomethyl)-[-3-(p-tert.-butylphenyl)]-pyrrolidin.

Die Guanidine I sind auf verschiedene Weise erhältlich, und zwar vorzugsweise nach den folgenden Methoden:

a) Herstellung aus Thiuroniumsalzen und Aminen

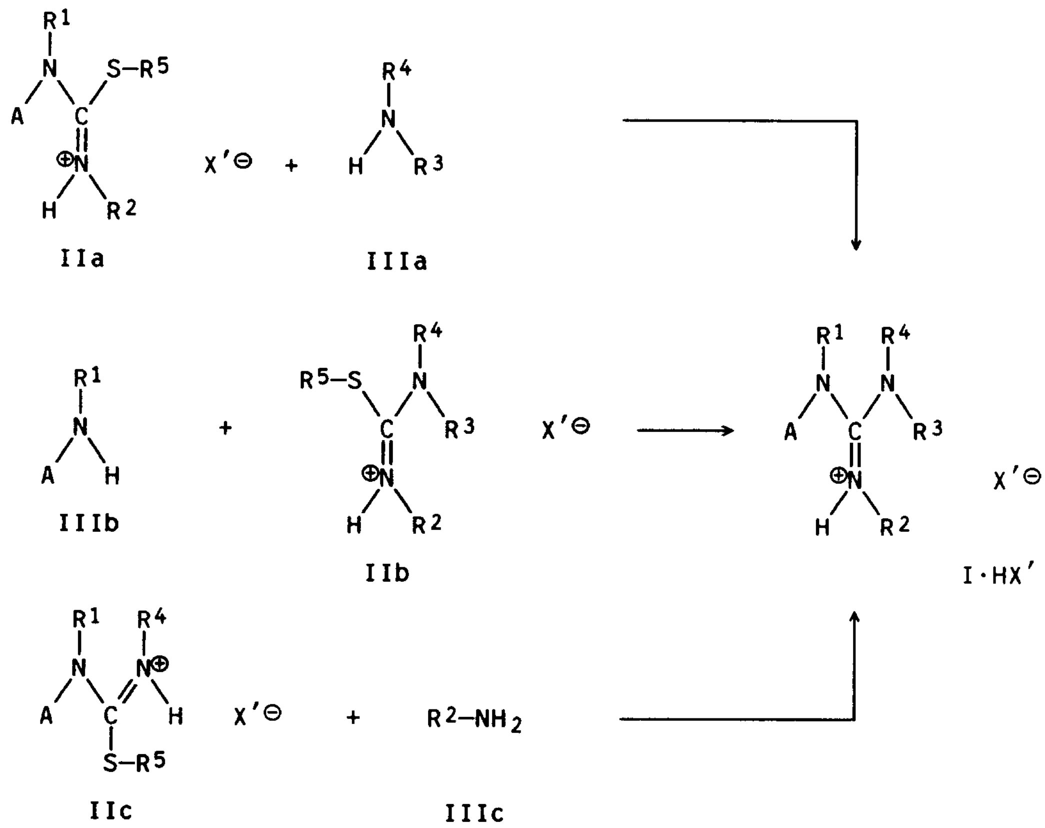

IIa IIIa IIIb IIb I·HX' IIc IIIc

In diesen Formeln bedeutet $R^5$ eine Benzylgruppe oder einen kurzkettigen Alkylrest, z.B. Methyl oder Ethyl, und X' vorteilhaft Chlorid, Bromid, Iodid, Sulfat, Methylsulfat, Methylsulfonat oder Tosylat.

Die Ausgangsverbindungen IIa-IIc und IIIa-IIIc sind bekannt oder auf bekannte Weise erhältlich, wobei bezüglich der Thiuroniumsalze auf Houben-Weyl, Methoden der Organischen Chemie, 4. Auflage, Bd. IX, S. 900ff. verwiesen sei.

Die aus Houben-Weyl, Methoden der Organischen Chemie, 4. Auflage, Bd. VIII, S. 1893f und Bd. E4, S. 614f an sich bekannte Umsetzung von Thiuroniumsalzen mit Aminen zu den Guanidinderivaten I•HX' erfolgt bevorzugt in polaren Lösungsmitteln wie Alkoholen, Ketonen, Ethern, Nitrilen, Dimethylsulfoxid, N-Methylpyrrolidon, Dimethylformamid oder Dimethylacetamid.

Man kann die Mengenverhältnisse der Reaktionspartner je nach den eingesetzten Verbindungen variieren. Vorteilhaft setzt man äquimolare Mengen um oder arbeitet besonders bevorzugt mit der doppelten benötigten Menge der Aminkomponente. Zusätzlich kann auch ein tertiäres Amin wie Triethylamin als Hilfsbase zum Abfangen des entstehenden Mercaptans zugegeben werden. Bevorzugt setzt man in diesem Fall äquimolare Mengen der Hilfsbase, bezogen auf das Thiuroniumsalz, ein.

Für die Umsetzungen empfehlen sich Temperaturen zwischen 20 °C und der Siedetemperatur des Lösungsmittels, vorzugsweise zwischen 60 und 130 °C. Da die Reaktionen nicht druckabhängig sind, arbeitet man vorzugsweise bei Normaldruck.

Durch Anionenaustausch lassen sich Salze mit anderen Anionen $X^\ominus$ oder - bei Ersatz durch Hydroxyl-Ionen - die freien Basen I erhalten.

b) Herstellung aus Aminoiminomethansulfonsäuren und Aminen

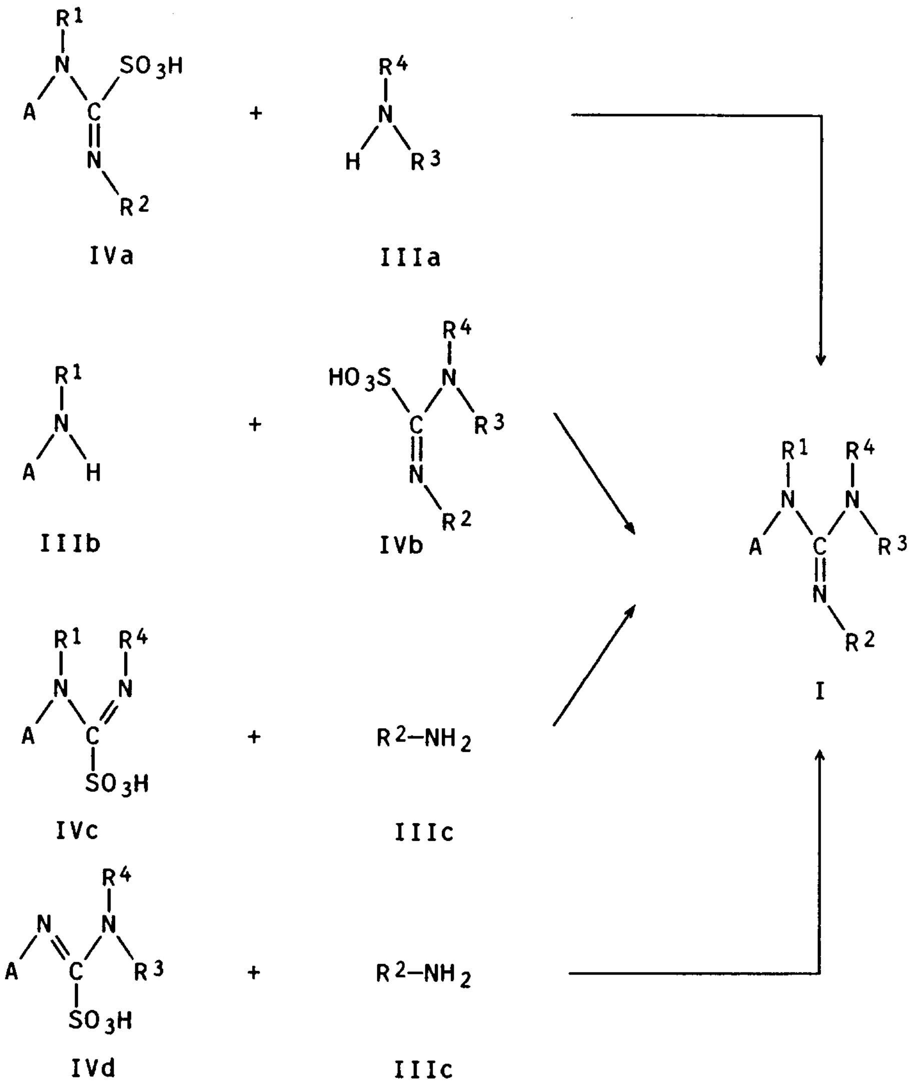

IVa IIIa

IIIb IVb

I

IVc IIIc

IVd IIIc

Die Ausgangsverbindungen IVa-IVd sind bekannt bzw. auf bekannte Weise aus Thioharnstoffderivaten erhältlich (z.B. C.A. Maryanoff et al., J. Org. Chem., Band 51, 1986, S. 1882f.).

Die aus C.A. Maryanoff et al., dto., an sich bekannte Umsetzung von Aminoiminosulfonsäuren mit Aminen zu den Guanidinderivaten I erfolgt bevorzugt in polaren Lösungsmitteln wie Alkoholen oder besonders bevorzugt in Acetonitril.

Für die Umsetzungen empfehlen sich Temperaturen zwischen 0 ° C und der Siedetemperatur des Lösungsmittels.

Bezüglich der Mengenverhältnisse und des Druckes gelten die Angaben für Methode a).

c) Herstellung aus Carbodiimiden und Aminen

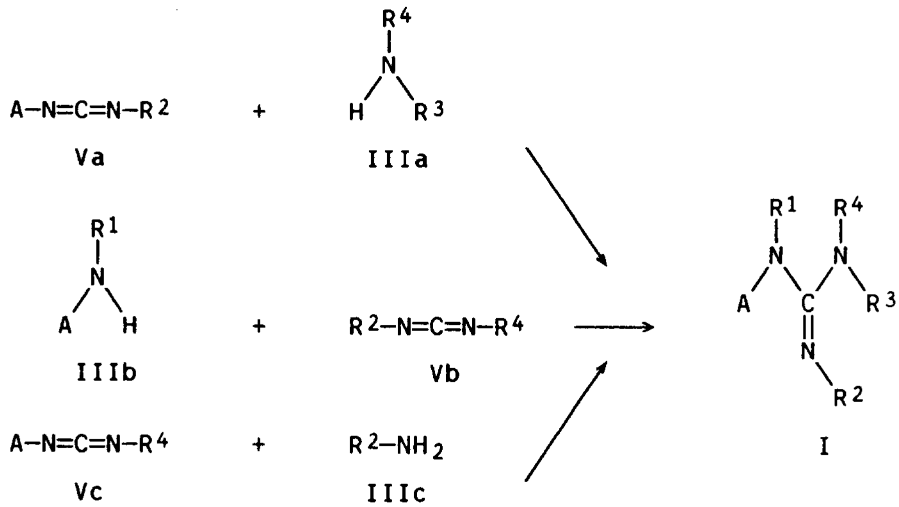


Die Ausgangsverbindungen Va-Vc sind bekannt bzw. nach bekannten Verfahren erhältlich. Beispielhaft sei auf M. Mikolaiczyk, Tetrahedron, Band 37, 1981, S. 233ff., Z.M. Jászay et al, Synthesis, 1987, S. 520ff. und auf G. Appel et al., Chem. Ber., Band 104, 1971, S. 1335f. verwiesen.

Die aus Houben-Weyl, Methoden der Organischen Chemie, 4. Auflage, Bd. VIII, S. 180 und Bd. E4, S. 609 an sich bekannte Umsetzung von Carbodiimiden mit Aminen zu den Guanidinderivaten I erfolgt bevorzugt in unpolaren Lösungsmitteln wie Hexan, Toluol, in kurzkettigen Alkoholen wie Methanol oder Isopropanol oder in Nitrilen wie Acetonitril.

Für die Umsetzungen empfehlen sich äquimolare Mengen an Ausgangsverbindungen oder bevorzugt ein geringer Überschuß der Aminkomponente, bis etwa 10 %.

Bezüglich der Temperatur und des Druckes gelten die Angaben für Methode a).

d) Herstellung aus Diphenylimidocarbonaten und Aminen

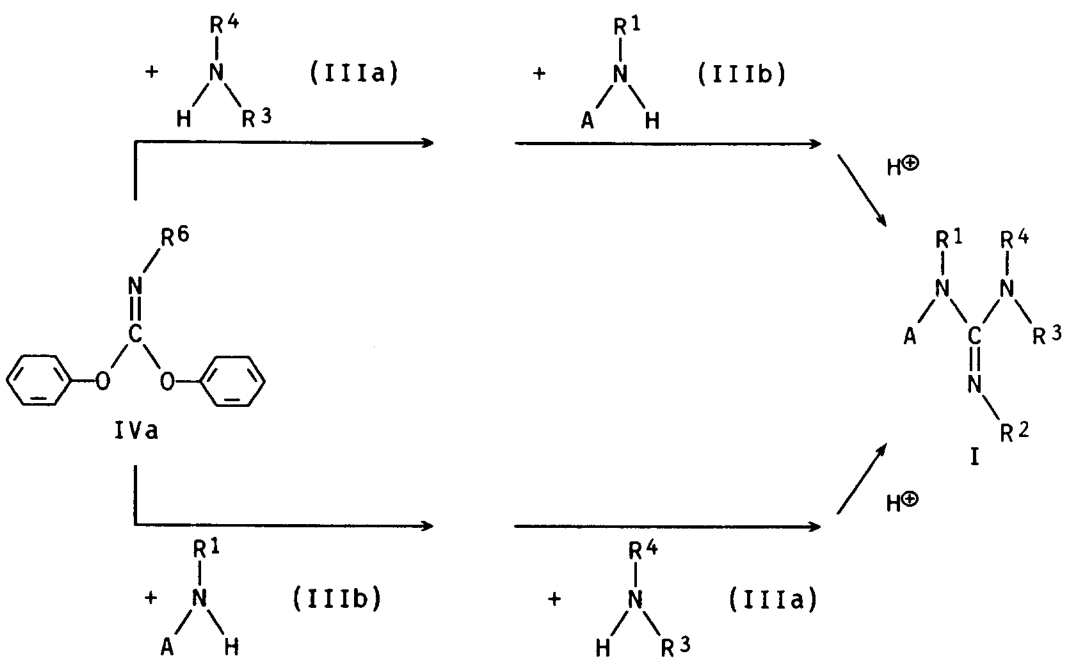


in der Formel IVa bedeutet $R^6$ eine Cyan-, Benzoyl- oder Methansulfonylgruppe.

Die aus A. Buschauer, Arzneim.-Forsch./Drug. Res., Band 37 (II), 1987, S. 1003/1008ff., und Arch. Pharm., Band 321, 1988, S. 281 an sich bekannte Umsetzung von Diphenylimidocarbonaten mit zwei Aminen erfolgt in zwei getrennten Stufen. Die Reaktion des Imidocarbonats mit dem ersten Amin wird bevorzugt in einem chlorierten Kohlenwasserstoff wie Methylenchlorid, einem Ether wie Tetrahydrofuran

oder Diethylether oder in einem Nitril wie Acetonitril durchgeführt. Die weitere Umsetzung des Produktes mit dem 2. Amin erfolgt in einem polaren Lösungsmittel wie Acetonitril oder Pyridin.

Bezüglich der Mengenverhältnisse, der Temperatur und des Druckes gelten die Angaben für Methode c).

Die Hydrolyse der erhaltenen Basen I mit $R^2 = R^6$ erfolgt auf bekannte Weise, vorteilhaft in einer Mineralsäure bei z.B. 70 bis 120 °C. Bevorzugt verwendet man 2 bis 12,5 M Salzsäure unter Rückfluß.

Man erhält die Chlorid-Salze der erfindungsgemäßen Verbindungen I mit $R^2$ = Wasserstoff.

e) Herstellung aus Bromcyan und Aminen

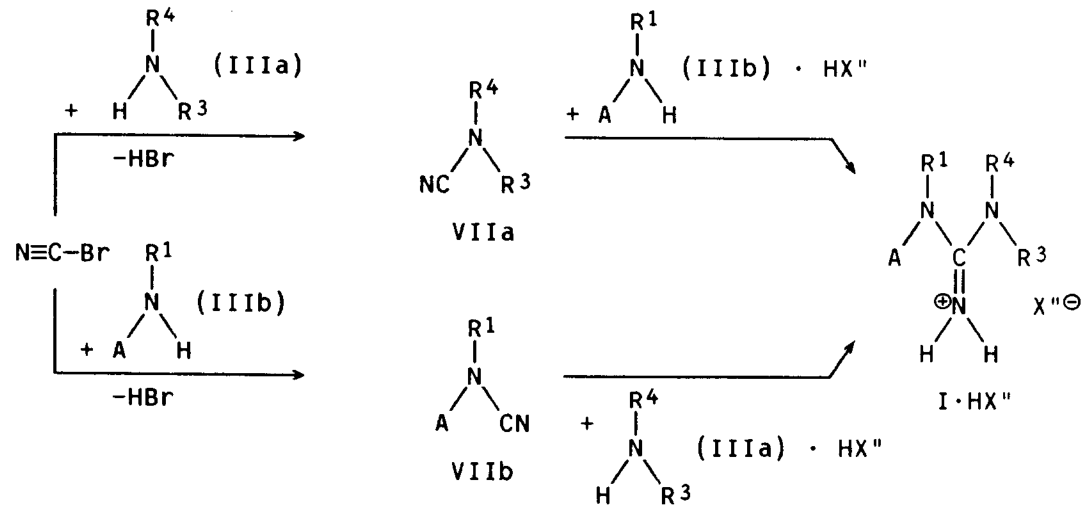


In diesen Formeln bedeutet X" bevorzugt Chlorid.

Die aus H.W. Geluk et al., J. Med. Chem., Band 12, 1969, S. 712f. an sich bekannte Umsetzung von Bromcyan mit Aminen zu N-substituierten Cyanamid-Derivaten VIIa oder VIIb erfolgt bevorzugt in Ethern wie Diethylether oder Tetrahydrofuran.

Vorteilhaft arbeitet man mit einem Überschuß der Aminkomponente gegenüber dem Bromcyan, bis zu etwa 60 %.

Für die Umsetzung empfehlen sich Temperaturen zwischen 0 und 25 °C. Da die Reaktionen nicht druckabhängig sind, arbeitet man vorzugsweise bei Normaldruck.

Die Umsetzung der N-substituierten Cyanamide VIIa und VIIb mit den Hydrochloriden der Amine IIIb bzw. IIIa erfolgt bevorzugt lösungsmittelfrei, bei Temperaturen von beispielsweise 150 bis 250 °C.

Bezüglich der eingesetzten Mengen, des Druckes oder der Darstellung der freien Basen I gelten die Angaben für Methode c).

Die Verbindungen der Formel I und ihre definitionsgemäßen Salze und Metallkomplexe eignen sich als Fungizide bei guter Pflanzenverträglichkeit.

Die erfindungsgemäßen Guanidine zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,

Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Besonders bevorzugt ist die Verwendung der Guanidine I gegen Botrytis cinerea.

Die Verbindungen werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung des Guaninidins gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 6 kg, insbesondere 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

- VI. eine innige Mischung aus 3 Gew.-Teilen der Verbindung Nr. 1b und 97 Gew.-Teilen feinteiligem Kaolin; dieses Stäubemittel enthält 3 Gew.-% Wirkstoff;
- VII. eine innige Mischung aus 30 Gew.-Teilen der Verbindung Nr. 2b, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
- VIII. eine stabile wäßrige Dispersion aus 40 Gew.-Teilen der Verbindung Nr. 4b, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
- IX. eine stabile ölige Dispersion aus 20 Gew.-Teilen der Verbindung Nr. 9b, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Herstellungsbeispiele

Beispiel A, nach Methode a) (Tabellenbeispiel 7b)

N-1-(4-tert.-Butylbenzyl)-N-2-methyl-N-3-(3,5,5-trimethyl-hexyl)-guanidin-hydroiodid

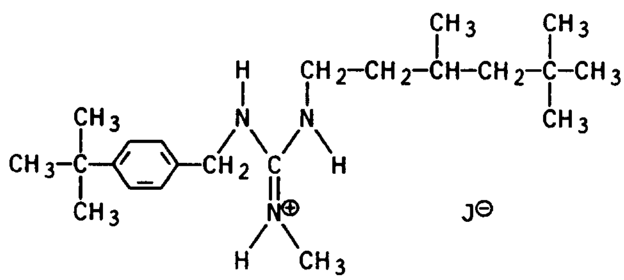

Eine Mischung aus 19 g (0,05 mol) N-(4-tert.-Butylbenzyl)-N',S-dimethyl-isothiuroniumiodid, 14,1 g (0,1 mol) 3,5,5-Trimethylhexylamin, 5 g (0,05 mol) Triethylamin und 20 g Molekularsieb (4Å) in 200 ml wasserfreiem Acetonitril wurde unter einer Stickstoffatmosphäre 6 Stunden lang unter Abspaltung von Methanthiol auf Rückflußtemperatur erhitzt. Nach heißer Filtration wurde wie üblich aufgearbeitet.
Ausbeute: 64 % d.Th.: Fp.: 165 ° C.

Vorstufe A1

N-(4-tert.-Butylbenzyl)-N'-methylthioharnstoff

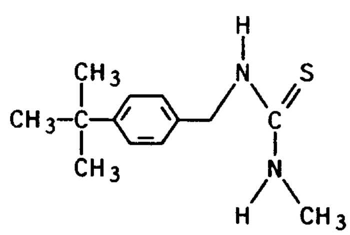

Bei 20 bis 30 ° C wurde eine Lösung von 163 g (1 mol) 4-tert.-Butylbenzyl-amin in 100 ml wasserfreiem Acetonitril unter Kühlung zu einer Mischung aus 73 g (1 mol) Methylisothiocyanat in 80 ml wasserfreiem Acetonitril getropft. Nach 3-stündigem Rühren bei 20 ° C und einer weiteren Stunde bei 80 bis 90 ° C wurde die Lösung eingeengt, das Rohprodukt mit Hexan aus Essigsäureethylester umgefällt, mit Hexan gewaschen und im Vakuum bei 60 ° C getrocknet.
Ausbeute: 72 % d.Th.; Fp 72 ° C.

Vorstufe A2

N-(4-tert.-Butylbenzyl)-N',S-dimethyl-isothiuroniumiodid

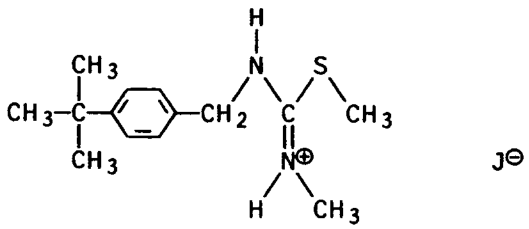

Eine Lösung von 23,6 g (0,1 mol) N-(4-tert.-Butylbenzyl)-N'-methylthioharnstoff und 114,2 g (0,1 mol) Iodmethan in 200 ml Methanol wurde 1 Stunde auf Rückflußtemperatur erhitzt. Nach dem Abkühlen auf Raumtemperatur wurde das Produkt durch Zugabe von Methyl-tert.-butylether ausgefällt, gewaschen und im Vakuum bei 40 ° C getrocknet.
Ausbeute: 53 % d. Th.; Fp: 172 ° C.

Beispiel B, nach Methode a) (Tabellenbeispiel 20b)

N-1-(4-tert.-Butylbenzyl)-N-2-n-octyl-guanidin-hydroiodid

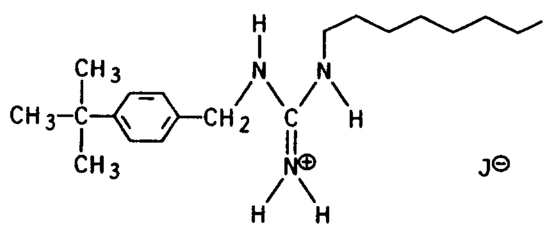

Eine Mischung aus 9,1 g ($2{,}5 \cdot 10^{-2}$ mol) N-(4-tert.-Butylbenzyl)-S-methyl-isothiuroniumiodid, 6,5 g ($5 \cdot 10^{-2}$ mol) n-Octylamin, 2,5 g (25 mmol) Triethylamin, 3 g Molekularsieb (4 Å) und 200 ml Acetonitril wurde 48 Stunden auf Rückflußtemperatur erhitzt und wie in Beispiel A beschrieben aufgearbeitet.
Ausbeute: 61 % d. Th.; Fp.: 113 ° C.

Vorstufe B1

N-(4-tert.-Butylbenzyl)-thioharnstoff

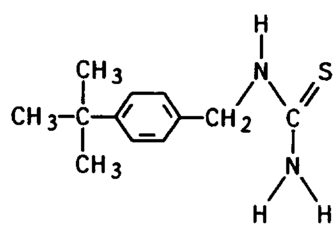

Zu einer Lösung von 82,5 g (1,1 mol) Ammoniumrhodanid in 300 ml absoluten Aceton wurden innerhalb von 10 Minuten 147,5 g (1,05 mol) Benzoylchlorid getropft. Nach 10 Minuten Rühren bei Rückflußtemperatur wurde eine Lösung von 163 g (1 mol) 4-tert.-Butylbenzylamin in 150 ml Aceton zugetropft, weitere 20 Minuten zum Rückfluß erhitzt und das Reaktionsgemisch in 2 l Eiswasser eingerührt. Der ausgefallene

Feststoff wurde mit Wasser gewaschen, in einem heißen Gemisch aus 1 l 10 %iger Natronlauge und 550 ml Ethanol gelöst und weitere 15 Minuten zum Rückfluß erhitzt.

Die anschließend mit Eiswasser verdünnte Mischung wurde mit konz. Salzsäure auf pH = 1 und anschließend mit festem Natriumhydrogencarbonat auf ca. pH = 9 eingestellt. Der resultierende Niederschlag wurde mit Wasser gewaschen und bei 60 °C im Vakuum getrocknet.
Ausbeute: 92 % d. Th.; Fp.: 80 °C.

Vorstufe B2

N-(4-tert.-Butylbenzyl)-S-methyl-isothiuroniumiodid

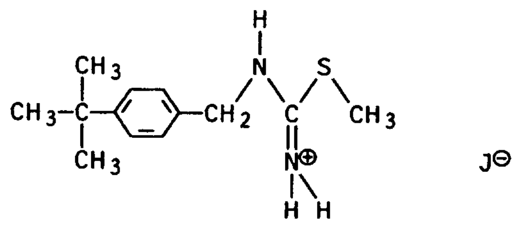

Zu 85 g (0,383 mol) N-(4-tert.-Butylbenzyl)-thioharnstoff in 200 ml Methanol wurden bei 30 bis 40 °C 54,4 g (0,333 mol) Iodmethan getropft. Umsetzung und Aufarbeitung erfolgten analog Beispiel A, Vorstufe A2; das Produkt wurde bei 60 °C getrocknet.
Ausbeute: 74 % d. Th.; Fp.: 143 °C.

Beispiel E, nach Methode c) (Tabellenbeispiel 11b)

N-1-(4-tert.-Butylbenzyl)-N-2-n-butyl-N-3-(2,2-Dimethylpropyl)-guanidin-hydrochlorid

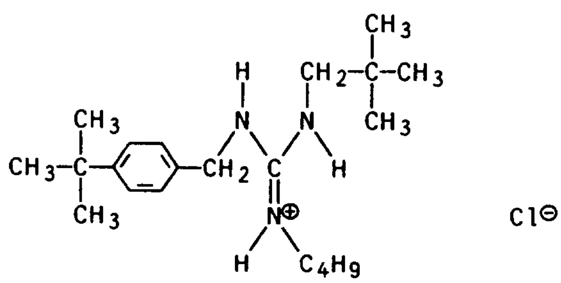

Eine Mischung aus 2 g ($8{,}2 \cdot 10^{-3}$ mol) N-(4-tert.-Butylbenzyl)-N'-n-butyl-carbodiimid, 0,17 g ($8{,}2 \cdot 10^{-3}$ mol) 2,2-Dimethylpropylamin und 100 ml wasserfreiem tert.-Butanol wurde 24 Stunden lang auf Rückflußtemperatur erhitzt. Nach Isolierung wurde das ölige Produkt mit methanolischer Salzsäure versetzt, im Vakuum aufkonzentriert und durch Verreiben mit Methyl-tert.-butylether zur Kristallisation gebracht.
Ausbeute: 33 % d. Th.; Fp.: 88 °C.

Analog den Beispielen A bis E wurden die in der folgenden Tabelle aufgeführten Verbindungen hergestellt:

Tabelle

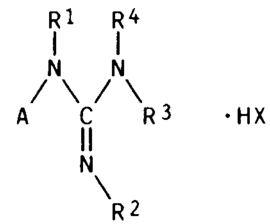

| Verb.-Nr. | A | R1 | R2 | R3 | R4 | HX | Fp./IR (Film) [$cm^{-1}$] |
|---|---|---|---|---|---|---|---|
| 1b | p-tert.-Butyl-benzyl | H | $CH_3$ | H | 3-(4-tert.-Butylphenyl)-2-methylpropyl | HCl | 125°C |
| 2b | p-tert.-Butyl-benzyl | H | $CH_3$ | R3 + R4 = 4-tert.-Butyl-piperidinyl | | HJ | 155°C |
| 3b | p-tert.-Butyl-benzyl | H | $CH_3$ | H | 4-tert.-Butylbenzyl | - | 2963, 2904, 2867, 1863, 1637, 1513, 1476, 1464 |
| 4b | p-tert.-Butyl-benzyl | H | $CH_3$ | H | 4-tert.-Butylbenzyl | HCl | 125°C |
| 5b | p-tert.-Butyl-benzyl | H | $CH_3$ | H | 4-tert.-Butylbenzyl | $CH_3COOH$ | 2963, 2905, 1638, 1568, 1514, 1477, 1393, 1365 |
| 6b | p-tert.-Butyl-benzyl | H | $CH_3$ | H | Cyclohexylmethyl | HJ | 186°C |
| 7b | p-tert.-Butyl-benzyl | H | $CH_3$ | H | 3,5,5-Trimethylhexyl | HJ | 165°C |
| 8b | Benzyl | H | n-Butyl | H | 3-(4-tert.-Butylphenyl)-2-methylpropyl | $CH_3COOH$ | 2958, 2928, 2869, 1639, 1510, 1495, 1453, 1362 |
| 9b | p-tert.-Butyl-benzyl | H | n-Butyl | R3 + R4 = cis-2,6-dimethyl-morpholin-4-yl | | $CH_3COOH$ | 2963, 2935, 2872, 1627, 1590, 1269, 1087 |
| 10b | p-tert.-Butyl-benzyl | H | n-Butyl | R3 + R4 = cis-2,6-dimethyl-morpholin-4-yl | | HCl | 60°C |
| 11b | p-tert.-Butyl-benzyl | H | n-Butyl | H | 2,2-Dimethylpropyl | HCl | 88°C |

| Verb.-Nr. | A | $R^1$ | $R^2$ | $R^3$ | $R^4$ | HX | Fp./IR (Film)[$cm^{-1}$] |
|---|---|---|---|---|---|---|---|
| 12b | p-tert.-Butyl-benzyl | H | n-Butyl | H | 2,2-Dimethylpropyl | $CH_3COOH$ | 2962, 2872, 1630, 1574, 1394, 1366 |
| 13b | p-tert.-Butyl-benzyl | H | n-Butyl | H | 4-tert.-Butylbenzyl | $CH_3COOH$ | 2962, 2906, 1634, 1570, 1394 |
| 14b | p-tert.-Butyl-benzyl | H | n-Butyl | H | 4-tert.-Butylbenzyl | HCl | 102°C |
| 15b | p-tert.-Butyl-benzyl | H | n-Butyl | H | 3-(4-tert.-Butylphenyl)-2-methylpropyl | HCl | 95 bis 100°C |
| 16b | p-tert.-Butyl-benzyl | H | n-Butyl | H | 3-(4-tert.-Butylphenyl)-2-methylpropyl | $CH_3COOH$ | 2962, 2933, 1633, 1571, 1394, 1364 |
| 17b | p-tert.-Butyl-benzyl | H | n-Butyl | H | n-Pentyl | - | |
| 18b | p-tert.-Butyl-benzyl | H | H | H | n-Hexyl | HJ | 156°C |
| 19b | p-tert.-Butyl-benzyl | H | H | H | n-Heptyl | HJ | 135°C |
| 20b | p-tert.-Butyl-benzyl | H | H | H | n-Octyl | HJ | 113°C |
| 21b | p-tert.-Butyl-benzyl | H | H | H | n-Nonyl | HJ | 3247, 3179, 2956, 2924, 2854, 1649, 1629, 1465 |
| 22b | p-tert.-Butyl-benzyl | H | H | H | n-Decyl | HJ | 3248, 3179, 2957, 2925, 2855, 1649, 1630, 1466 |
| 23b | p-tert.-Butyl-benzyl | H | H | H | n-Dodecyl | HJ | 3247, 3180, 2957, 2924, 2854, 1649, 1630, 1466 |
| 24b | p-tert.-Butyl-benzyl | H | $CH_3$ | H | n-Hexyl | HJ | 177°C |
| 25b | p-tert.-Butyl-benzyl | H | $CH_3$ | H | n-Octyl | HJ | 122°C |

| Verb.-Nr. | A | $R^1$ | $R^2$ | $R^3$ | $R^4$ | HX | Fp./IR (Film)[$cm^{-1}$] |
|---|---|---|---|---|---|---|---|
| 26b | p-tert.-Butyl-benzyl | H | $CH_3$ | H | n-Decyl | HJ | 85°C |
| 27b | p-tert.-Butyl-benzyl | H | $CH_3$ | H | n-Dodecyl | HJ | 96°C |
| 28b | p-tert.-Butyl-benzyl | H | $CH_3$ | H | 2,2-Dimethylpropyl | HJ | 165°C |
| 29b | p-tert.-Butyl-benzyl | H | $CH_3$ | H | 3-Methyl-butyl | HJ | 137°C |
| 30b | p-tert.-Butyl-benzyl | H | $CH_3$ | $R^3$ + $R^4$ = Piperidinyl | | HJ | 82°C |
| 31b | p-tert.-Butyl-benzyl | H | $CH_3$ | H | Phenethyl | HJ | 87°C |
| 32b | p-tert.-Butyl-benzyl | H | $CH_3$ | H | Benzyl | HJ | 124°C |
| 33b | p-tert.-Butyl-benzyl | H | $CH_3$ | H | 3-(4-tert.-Butylphenyl)-2-methylpropyl | HJ | 165°C |
| 34b | p-tert.-Butyl-benzyl | H | $CH_3$ | H | 2-Ethyl-hexyl | HJ | 65°C |
| 35b | p-tert.-Butyl-benzyl | H | $CH_3$ | H | n-Tridecyl | HJ | 105°C |
| 36b | p-tert.-Butyl-benzyl | H | $CH_3$ | H | 4-Methoxy-phenethyl | HJ | 137°C |
| 37b | p-tert.-Butyl-benzyl | H | $CH_3$ | H | 6-Ethyl-4-oxa-decyl | HJ | 3218, 2959, 2929, 2870, 1623, 1462, 1380, 1109 |
| 38b | p-tert.-Butyl-benzyl | H | $CH_3$ | H | 4-Phenyl-but-2-yl | HJ | 102°C |
| 39b | p-tert.-Butoxy-benzyl | H | H | H | 4-tert.-Butyl-cyclo-hexylmethyl | HJ | 192°C |

| Verb.-Nr. | A | $R^1$ | $R^2$ | $R^3$ | $R^4$ | HX | Fp./IR (Film)[$cm^{-1}$] |
|---|---|---|---|---|---|---|---|
| 43b | p-tert.-Butyl-benzyl | H | H | H | 3-Diethylaminopropyl | HJ | 3243, 3179, 2964, 2870, 2822, 1629, 1465, 1366 |
| 44b | p-tert.-Butyl-benzyl | H | H | H | 4-Methoxy-phenethyl | HJ | 3303, 3185, 3164, 1643, 1632, 1595, 1512, 1242 |
| 45b | p-tert.-Butyl-benzyl | H | H | H | Phenethyl | HJ | 130°C |
| 46b | p-tert.-Butyl-benzyl | H | H | H | 2-Ethylhexyl | HJ | 3251, 3181, 2960, 2929, 2872, 1649, 1630, 1464 |
| 47b | p-tert.-Butyl-benzyl | H | H | H | Benzyl | HJ | 58°C |
| 48b | p-tert.-Butyl-benzyl | H | H | $R^3$ + $R^4$ = 4-tert.-Butyl-piperidinyl | | HJ | 183°C |
| 49b | p-tert.-Butyl-benzyl | H | H | H | 4-tert.-Butylbenzyl | HJ | 182°C |
| 50b | p-tert.-Butyl-benzyl | H | H | H | 3-(4-tert.-Butylphenyl)-2-methylpropyl | HJ | 152°C |
| 51b | p-tert.-Butyl-benzyl | H | H | H | Tridecyl | HJ | 89°C |
| 52b | p-tert.-Butyl-benzyl | H | H | H | 6-Ethyl-4-oxa-decyl | HJ | 100°C |
| 53b | p-tert.-Butyl-benzyl | H | H | $R^3$ + $R^4$ = Piperidinyl | | HJ | 72°C |

| Verb.-Nr. | A | $R^1$ | $R^2$ | $R^3$ | $R^4$ | HX | Fp./IR (Film)[$cm^{-1}$] |
|---|---|---|---|---|---|---|---|
| 54b | p-tert.-Butyl-benzyl | H | $CH_3$ | H | 4-tert.-Butyl-cyclo-hexylmethyl | HJ | 125°C |
| 55b | p-tert.-Butyl-benzyl | H | $CH_3$ | H | 3-Diethylaminopropyl | HJ | 3210, 3102, 2964, 2870, 1620, 1458, 1383, 1364 |
| 56b | p-tert.-Butyl-benzyl | H | $CH_3$ | H | 1-Phenyl-ethyl | HJ | 163°C |
| 57b | p-(1,1-Dimethyl-ethyl)-benzyl | H | H | H | n-Hexyl | - | |
| 58b | p-(1,1-Dimethyl-ethyl)-benzyl | H | H | H | n-Heptyl | - | |
| 59b | p-(1,1-Dimethyl-ethyl)-benzyl | H | H | H | n-Octyl | - | |
| 60b | p-(1,1-Dimethyl-ethyl)-benzyl | H | H | H | n-Nonyl | - | |
| 61b | p-(1,1-Dimethyl-ethyl)-benzyl | H | H | H | tert.-Butylbenzyl | - | |
| 62b | p-(1,1-Dimethyl-ethyl)-benzyl | H | H | H | 3-Methylbutyl | - | |
| 63b | p-(1,1-Dimethyl-ethyl)-benzyl | H | H | H | 3-(4-tert.-Butylphenyl)-2-methylpropyl | - | |
| 64b | p-tert.-Butoxy-benzyl | H | H | H | n-Hexyl | - | |
| 65b | p-tert.-Butoxy-benzyl | H | H | H | n-Heptyl | - | |
| 66b | p-tert.-Butoxy-benzyl | H | H | H | n-Octyl | - | |
| 67b | p-tert.-Butoxy-benzyl | H | H | H | n-Nonyl | - | |

| Verb.-Nr. | A | $R^1$ | $R^2$ | $R^3$ | $R^4$ | HX | Fp./IR (Film)[$cm^{-1}$] |
|---|---|---|---|---|---|---|---|
| 68b | p-tert.-Butoxybenzyl | H | H | H | n-Decyl | - | |
| 69b | p-tert.-Butoxybenzyl | H | H | H | 3-Methylbutyl | - | |
| 70b | p-tert.-Butoxybenzyl | H | H | H | tert.-Butylbenzyl | - | |
| 71b | p-tert.-Butoxybenzyl | H | H | H | tert.-Butoxybenzyl | - | |
| 72b | p-tert.-Butoxybenzyl | H | H | H | n-Octyl | - | |
| 73b | p-(1,1,2-Trimethylpropyl)-benzyl | H | H | H | n-Hexyl | - | |
| 74b | p-(1,1,2-Trimethylpropyl)-benzyl | H | H | H | n-Heptyl | - | |
| 75b | p-(1,1,2-Trimethylpropyl)-benzyl | H | H | H | n-Octyl | - | |
| 76b | p-(1,1,2-Trimethylpropyl)-benzyl | H | H | H | n-Nonyl | - | |
| 77b | p-(1,1,2-Trimethylpropyl)-benzyl | H | H | H | n-Decyl | - | |
| 78b | p-tert.-Butylbenzyl | H | $CH_3$ | H | Cyclohexylmethyl | HCl | 155°C |
| 79b | p-tert.-Butylbenzyl | H | $CH_3$ | H | Cyclohexylmethyl | $CH_3COOH$ | 82°C |

| Verb.-Nr. | A | $R^1$ | $R^2$ | $R^3$ | $R^4$ | HX | Fp./IR (Film)[$cm^{-1}$] |
|---|---|---|---|---|---|---|---|
| 80b | p-tert.-Butylbenzyl | H | $CH_3$ | H | Cyclohexylmethyl | $(COOH)_2$ | 168°C |
| 81b | p-tert.-Butylbenzyl | H | $CH_3$ | H | 3,5,5-Trimethylhexyl | $(COOH)_2$ | 180°C |
| 82b | p-tert.-Butylbenzyl | H | $CH_3$ | H | 3,5,5-Trimethylhexyl | $CH_3COOH$ | 75°C |
| 83b | p-tert.-Butylbenzyl | H | $CH_3$ | H | 3,5,5-Trimethylhexyl | HCl | 107°C |
| 84b | p-tert.-Butylbenzyl | H | $CH_3$ | H | n-Hexyl | HCl | 105°C |
| 85b | p-tert.-Butylbenzyl | H | $CH_3$ | H | n-Hexyl | $CH_3COOH$ | 110°C |
| 86b | p-tert.-Butylbenzyl | H | $CH_3$ | H | n-Hexyl | $(COOH)_2$ | 185°C |
| 87b | p-tert.-Butylbenzyl | H | $CH_3$ | H | 3-Methylbutyl | $(COOH)_2$ | 182°C |
| 88b | p-tert.-Butylbenzyl | H | H | H | 3-Methylbutyl | $CH_3COOH$ | 72°C |
| 89b | p-tert.-Butylbenzyl | H | H | H | 3-Methylbutyl | HCl | 105°C |
| 90b | p-tert.-Butylbenzyl | H | H | H | 6-Ethyl-4-oxa-decyl | HCl | Öl, 3260, 3173, 2959, 2930, 2871, 1655, 1637, 1464 |
| 91b | p-tert.-Butylbenzyl | H | H | H | 6-Ethyl-4-oxa-decyl | $CH_3COOH$ | Öl, 2959, 2929, 2870, 1653, 1560, 1515, 1403, 1110 |
| 92b | p-tert.-Butylbenzyl | H | H | H | 6-Ethyl-4-oxa-decyl | $(COOH)_2$ | Öl, 3183, 2958, 2929, 2870, 1633, 1462, 1220, 1109 |
| 93b | p-tert.-Butylbenzyl | H | H | H | 4-tert.-Butoxybenzyl | $(COOH)_2$ | 118°C |

| Verb.-Nr. | A | $R^1$ | $R^2$ | $R^3$ | $R^4$ | HX | Fp./IR (Film)[$cm^{-1}$] |
|---|---|---|---|---|---|---|---|
| 94b | p-tert.-Butyl-benzyl | H | H | H | 4-tert.-Butoxybenzyl | $CH_3COOH$ | 168°C |
| 95b | p-tert.-Butyl-benzyl | H | $CH_3$ | H | 3-(4-tert.-Butylphenyl)-2-methylpropyl | $CH_3COOH$ | 97°C |
| 96b | p-tert.-Butyl-benzyl | H | $CH_3$ | H | 3-(4-tert.-Butylphenyl)-2-methylpropyl | $H_3BO_3$ | 149°C |
| 97b | p-tert.-Butyl-benzyl | H | $CH_3$ | H | 3-(4-tert.-Butylphenyl)-2-methylpropyl | - | 2960, 2903, 2867, 1636, 1512, 1474, 1462, 1362 |
| 98b | p-tert.-Butyl-benzyl | H | H | H | 2-Ethylhexyl | HCl | 80°C |
| 99b | p-tert.-Butyl-benzyl | H | H | H | 2-Ethylhexyl | $CH_3COOH$ | 2959, 2928, 2870, 1569, 1462, 1402, 1363, 1269 |
| 100b | p-tert.-Butyl-benzyl | H | H | $R^3$+$R^4$ = | 4-tert.-Butyl-piperidinyl | HCl | 250°C |
| 101b | p-tert.-Butyl-benzyl | H | H | $R^3$+$R^4$ = | 4-tert.-Butyl-piperidinyl | $CH_3COOH$ | |
| 102b | p-tert.-Butyl-benzyl | H | H | H | 3-(4-tert.-Butylphenyl)-2-methylpropyl | HCL | 90°C |
| 103b | p-tert.-Butyl-benzyl | H | H | H | 3-(4-tert.-Butylphenyl)-2-methylpropyl | $CH_3COOH$ | 3278, 3198, 2961, 1635, 1606, 1561, 1516, 1409 |
| 104b | p-tert.-Butyl-benzyl | H | H | H | 4-Hydroxybenzyl | HCl | 3323, 3263, 3170, 2961, 1651, 1614, 1513, 1267 |
| 105b | p-tert.-Butyl-benzyl | H | H | H | 4-Hydroxybenzyl | $CH_3COOH$ | 3180, 3026, 2962, 1645, 1614, 1554, 1462, 1409 |
| 106b | p-tert.-Butyl-benzyl | H | H | $CH_3$ | 2,5,7,7-Tetramethyl-octyl | HJ | 58°C |
| 107b | p-tert.-Butyl-benzyl | H | $CH_3$ | H | 4-tert.-Butylbenzyl | HJ | 90°C |

| Verb.-Nr. | A | $R^1$ | $R^2$ | $R^3$ | $R^4$ | HX | Fp./IR (Film)[$cm^{-1}$] |
|---|---|---|---|---|---|---|---|
| 108b | p-tert.-Butyl-benzyl | H | H | $R^3+R^4$ = | 3-(1,5-Dimethylhexyl)-pyrrolidinyl | HJ | 172°C |
| 109b | p-tert.-Butyl-benzyl | H | H | $R^3+R^4$ = | 3-(1,5-Dimethylhexyl)-pyrrolidinyl | HCl | 190°C |
| 110b | p-tert.-Butyl-benzyl | H | H | $R^3+R^4$ = | 3-(1,5-Dimethylhexyl)-pyrrolidinyl | $CH_3COOH$ | 2955, 2928, 2868, 1610, 1573, 1465, 1448, 1402 |
| 111b | p-tert.-Butyl-benzyl | H | H | H | n-Undecyl | HJ | 3249, 3181, 2956, 2924, 2853, 1649, 1629, 1464 |
| 112b | p-tert.-Butyl-benzyl | H | H | H | 6-Hydroxyhexyl | HJ | 3287, 3198, 2932, 2859, 1650, 1629, 1547, 1462 |
| 113b | p-tert.-Butyl-benzyl | H | H | H | 2-(2-Hydroxyethoxy)-ethyl | HJ | 3299, 3188, 2958, 2869, 1651, 1631, 1122, 1065 |
| 114b | p-tert.-Butyl-benzyl | H | H | H | 6-Hydroxy-6-methyl-hept-2-yl | HJ | 3297, 3188, 2964, 2867, 1626, 1602, 1556, 1462 |
| 115b | p-tert.-Butyl-benzyl | H | H | H | 6,10-Dimethyl-undec-2-yl | HJ | 3249, 3180, 2954, 2926, 2867, 1646, 1626, 1462 |
| 116b | p-tert.-Butyl-benzyl | H | H | H | 2,2-Dimethylpropyl | HJ | 85°C |
| 117b | p-tert.-Butyl-benzyl | H | H | H | 3-Methylbutyl | HJ | 122°C |
| 118b | p-tert.-Butyl-benzyl | H | $CH_3$ | H | n-Butyl | HJ | 135°C |
| 119b | p-tert.-Butyl-benzyl | H | $CH_3$ | H | n-Pentyl | HJ | 147°C |
| 120b | p-tert.-Butyl-benzyl | H | n-Butyl | H | cis-2,6-Dimethyl-morpholin-4-yl | - | 2962, 2931, 2904, 2869, 1637, 1375, 1144, 1086 |

| Verb.-Nr. | A | R1 | R2 | R3 | R4 | HX | Fp./IR (Film)[$cm^{-1}$] |
|---|---|---|---|---|---|---|---|
| 121b | p-tert.-Butyl-benzyl | H | n-Butyl | H | 2,2-Dimethylpropyl | - | 2956, 2904, 2867, 1649, 1513, 1476, 1464, 1362 |
| 122b | p-tert.-Butyl-benzyl | H | n-Butyl | H | 4-tert.-Butylbenzyl | - | 2959, 2931, 2904, 2868, 1633, 1513, 1463, 1362 |
| 123b | p-tert.-Butyl-benzyl | H | n-Butyl | H | 3-(4-tert.-Butylphenyl)-2-methylpropyl | - | 2960, 2928, 2869, 1640, 1512, 1462, 1362, 1269 |
| 124b | p-tert.-Butyl-benzyl | H | H | H | n-Butyl | HJ | 3248, 3182, 2959, 2932, 2870, 1649, 1630, 1463 |
| 125b | p-tert.-Butyl-benzyl | H | H | H | n-Pentyl | HJ | 120°C |

Anwendungsbeispiele

Als Vergleichswirkstoffe wurden N-1-(4-tert.-Butylphenyl)-N-2-cyclohexyl-N-3-allyl-guanidin (A), N-1-(4-tert.-Butylphenyl)-N-2-cyclohexyl-N-3-(3-N,N-dimethylamino-n-propyl)-guanidin (B) und N-1-(4-tert.-Butylphenyl)-N-2-cyclohexyl-N-3-(4-tert.butylcyclohexyl)-guanidin (C) gewählt, die aus der DE-A 31 08 564 bekannt sind.

Beispiel 1

Wirksamkeit gegen Pyrenophora teres

Gerstenkeimlinge der Sorte "Igri" wurden in Zweiblattstadium mit 0,05 %igen wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres infiziert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Danach wurde das Ausmaß des Pilzbefalls beurteilt.

Das Ergebnis zeigt, daß die Wirkstoffe 1b, 3b, 4b, 7b, 8b, 13b,14b, 15b, 16b, 18b, 20b, 22b, 23b, 25b, 26b, 34b, 37b, 52b und 54b bei der Anwendung als 0,05 %ige (Gew.-%) Spritzbrühe bessere fungizide Wirkungen zeigen (85 %) als die bekannten Vergleichswirkstoffe A, B und C (55 %).

Beispiel 2

Wirksamkeit gegen Weizenmehltau

Blätter von in Töpfen gewachsenen Weizenkeimlingen der Sorte "Kanzler" wurden mit 0,025 %iger wäßriger Spritzbrühe, die 80 % Wirkstoff und 20 % Emulgiermittel in der Trockenmasse enthielten, besprüht und 24 Stunden nach dem Antrocknen des Spritzbelages mit Sporen des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung beurteilt.

Das Ergebnis zeigt, daß die Wirkstoffe 2b, 6b, 9b, 11b, 12b, 13b, 29b, 31b, 34b, 35b, 36b, 38b, 45b und 46b bei der Anwendung als 0,025 %ige (Gew.-%) Spritzbrühe eine bessere fungizide Wirkung zeigen (85 %) als die bekannten Vergleichswirkstoffe B und C (60 %).

**Patentansprüche**

**1.** Substituierte Guanidine der allgemeinene Formel I

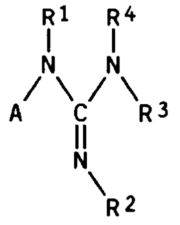

(I)

in der die Substituenten die folgende Bedeutung haben:

A eine Benzylgruppe, die in para-Position durch $C_1$-$C_{10}$-Alkyl oder $C_1$-$C_{10}$-Alkoxy substituiert ist, wobei der Substituent noch eine Hydroxyl- oder $C_1$-$C_6$-Alkoxygruppe tragen kann;

$R^1$,$R^2$,$R^3$ Wasserstoff oder $C_1$-$C_4$-Alkylgruppen;

$R^4$ eine $C_5$-$C_{18}$-Alkylgruppe, die durch Sauerstoff unterbrochen sein kann, eine $C_5$-$C_{18}$-Alkenylgruppe, eine $C_4$-$C_{18}$-Alkinylgruppe oder eine Phenyl-$C_1$-$C_6$-alkylgruppe, wobei diese Gruppen bis zu drei der folgenden Substituenten tragen können: Hydroxyl, Halogen, Cyan, $C_1$-$C_7$-Alkoxy oder bis zu zwei Amino-, $C_1$-$C_4$-Alkylamino- oder Di-$C_1$-$C_4$-alkylamino-Substituenten, und wobei der Phenylteil der Phenylalkylgruppe zusätzlich eine Phenoxygruppe oder bis zu 3 $C_2$-$C_4$-Alkenylgruppen, $C_1$-$C_4$-Alkoxy-$C_1$-$C_4$-alkylgruppen oder $C_1$-$C_6$-Alkylgruppen, die unsubstituiert oder partiell oder vollständig halogeniert sein können, tragen kann; oder eine $C_5$-$C_6$-Cycloalkyl-$C_1$-$C_8$-alkylgruppe, wobei der Cycloalkylring bis zu drei $C_1$-$C_4$-Alkylgruppen oder bis zu zwei Hydroxyl-

oder Trifluormethylgruppen tragen kann;

eine $C_3$-$C_4$-Alkylgruppe, die durch Sauerstoff unterbrochen sein kann oder eine $C_4$-Alkenylgruppe, wobei diese Gruppen bis zu 3 der folgenden Substituenten tragen können: Hydroxyl, Halogen, Cyan, $C_1$-$C_7$-Alkoxy oder bis zu zwei Amino-, $C_1$-$C_4$-Alkylamino- oder Di-($C_1$-$C_4$)-alkylaminogruppen; oder gemeinsam mit dem Rest $R^3$ und dem Stickstoffatom einen 5- oder 6-gliedrigen heterocyclischen Ring, der mit $C_1$-$C_6$-Alkyl, Phenyl, $C_1$-$C_6$-Alkylphenyl ein- bis dreifach substituiert und durch ein Sauerstoffatom unterbrochen sein kann;

sowie die pflanzenverträglichen mineralsauren Salze I•HX und Metallkomplexe von I.

**2.** Guanidine nach Anspruch 1, wobei die Substituenten folgende Bedeutung haben:

A: eine Benzylgruppe, die in para-Position durch $C_1$-$C_{10}$-Alkyl oder $C_1$-$C_{10}$-Alkoxy substituiert ist, wobei der Substituent noch eine Hydroxyl- oder $C_1$-$C_6$-Alkoxygruppe tragen kann;

$R^1$,$R^2$,$R^3$: Wasserstoff oder $C_1$-$C_4$-Alkylgruppen;

$R^4$: eine $C_3$-$C_{18}$-Alkylgruppe, die durch Sauerstoff unterbrochen sein kann, eine $C_4$-$C_{18}$-Alkenyl- oder eine $C_4$-$C_{18}$-Alkinylgruppe oder eine Phenyl-$C_1$-$C_6$-alkylgruppe, wobei diese Gruppen bis zu drei der folgenden Substituenten tragen können: Hydroxyl, Halogen, Cyan, $C_1$-$C_7$-Alkoxy oder bis zu zwei Amino-, $C_1$-$C_4$-Alkylamino- oder Di-($C_1$-$C_4$)-alkylamino-Substituenten, und wobei der Phenylteil der Phenylalkylgruppe zusätzlich eine Phenoxygruppe oder bis zu drei $C_2$-$C_4$-Alkenylgruppen, $C_1$-$C_4$-Alkoxy-$C_1$-$C_4$-alkylgruppen oder $C_1$-$C_6$-Alkylgruppen, die unsubstituiert oder partiell oder vollständig halogeniert sein können, tragen kann, oder eine $C_5$-$C_6$-Cycloalkyl-$C_1$-$C_8$-alkylgruppe, wobei der Cycloalkylring bis zu drei $C_1$-$C_4$-Alkylgruppen oder bis zu zwei Hydroxyl- oder Triflourmethylgruppen tragen kann; oder gemeinsam mit dem Rest $R^3$ und dem Stickstoffatom einen 5- oder 6-gliedrigen heterocyclischen Ring, der mit $C_1$-$C_6$-Alkyl, Phenyl oder $C_1$-$C_6$-Alkylphenyl ein- bis zweifach substituiert und durch ein Sauerstoffatom unterbrochen sein kann;

sowie die pflanzenverträglichen mineralsauren Salze I•HX und Metallkomplexe von I.

**3.** Verfahren zur Herstellung der Guanidine bzw. ihrer pflanzenverträglichen Salze gemäß Anspruch 1, dadurch gekennzeichnet, daß man entweder

a) ein Thiuronium-Salz IIa

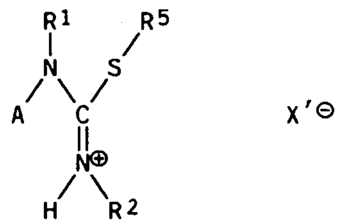

(IIa)

mit einem Amin IIIa

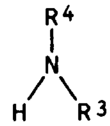

(IIIa)

oder ein Thiuronium-Salz IIb

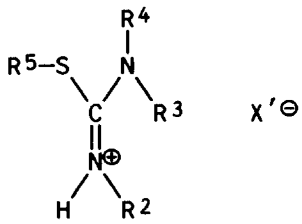

(IIb)

mit einem Amin IIIb

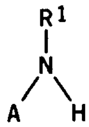

(IIIb)

oder ein Thiuronium-Salz IIc

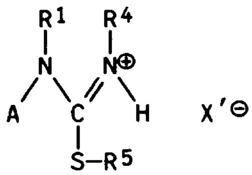

(IIc)

mit einem Amin IIIc

$R^2$-$NH_2$     IIIc

wobei $R^5$ eine Benzyl- oder $C_1$-$C_4$-Alkylgruppe bedeutet und x' für Halogen, Sulfat, Methylsulfat, $C_1$-$C_4$-Alkylsulfonat oder p-Toluylsulfonat steht, gewünschtenfalls in Gegenwart einer Hilfsbase umsetzt

oder
b) eine Aminoiminomethansulfonsäure IVa

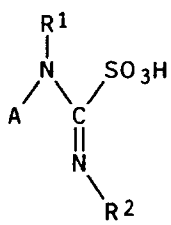

(IVa)

mit einem Amin IIIa,
oder eine Aminoiminomethansulfonsäure IVb

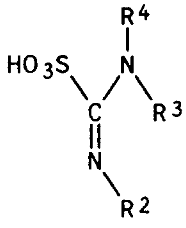

(IVb)

mit einem Amin IIIb,

oder eine Aminoiminomethansulfonsäure IVc oder IVd

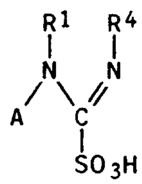 (IVc) 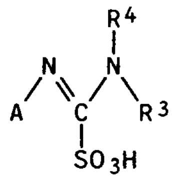 (IVd)

mit einem Amin IIIc umsetzt,
oder
c) ein Carbodiimid Va

$A\text{-}N=C=N\text{-}R^2$ (Va)

mit einem Amin IIIa,

oder ein Carbodiimid Vb

$R^2\text{-}N=C=N\text{-}R^4$ (Vb)

mit einem Amin IIIb,

oder ein Carbodiimid Vc

$A\text{-}N=C=N\text{-}R^4$ (Vc)

mit einem Amin IIIc umsetzt, oder
d) ein Diphenylimidocarbonat VIa

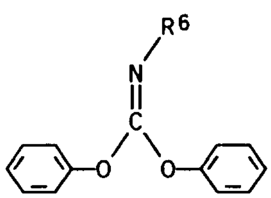

(VIa)

wobei $R^6$ eine Cyan-, Benzoyl- oder Methansulfonylgruppe bedeutet, stufenweise mit den beiden Aminen IIIa und IIIb umsetzt und anschließend hydrolysiert, oder
e) aus Bromcyan mit dem Amin IIIa das N-substiutierte Cyanamid VIIa

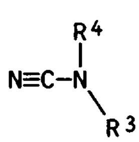

(VIIa)

herstellt und dieses anschließend mit dem mineralsauren Salz des Amins IIIb zu dem entsprechenden Salz der Verbindung I umsetzt,
oder mit Bromcyan aus dem Amin IIIb das N-substituierte Cyanamid VIIb

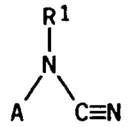

(VIIb)

herstellt und dieses anschließend mit dem mineralsauren Salz des Amins IIIa zu dem entsprechenden Salz der Verbindung I umsetzt,

und die erhaltenen Umsetzungsprodukte, soweit sie nicht bereits in Form der freien Basen oder der pflanzenverträglichen Salze anfallen, in die freien Basen bzw. die pflanzenverträglichen Salze überführt oder aus den freien Basen die Metallkomplexe herstellt.

4. Verwendung der Guanidine I, deren pflanzenverträglichen Salzen und Metallkomplexen gemäß Anspruch 1 als Fungizide.

5. Fungizides Mittel, enthaltend mindestens ein Guanidin der Formel I, dessen pflanzenverträgliches Salz oder dessen Metallkomplex gemäß Anspruch 1 und einen flüssigen oder festen Trägerstoff.

6. Verfahren zur Bekämpfung von Pilzen, dadurch gekennzeichnet, daß man eine fungizide wirksame Menge eines Guanidins der Formel I, dessen Salz oder Metallkomplex, gemäß Anspruch 1, auf Pilze, vom Pilzbefall bedrohte Pflanzen, deren Lebensraum oder auf das Saatgut der bedrohten Pflanzen einwirken läßt.

**Patentansprüche für folgende Vertragsstaaten : GR, ES**

1. Verfahren zur Herstellung substituierter Guanidine der allgemeinen Formel I

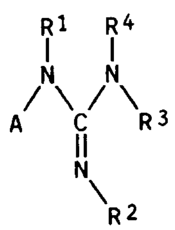

I

in der die Substituenten die folgende Bedeutung haben:

| | |
|---|---|
| A | eine Benzylgruppe, die in para-Position durch $C_1$-$C_{10}$-Alkyl oder $C_1$-$C_{10}$-Alkoxy substituiert ist, wobei der Substituent noch eine Hydroxyl- oder $C_1$-$C_6$-Alkoxygruppe tragen kann; |
| $R^1$,$R^2$,$R^3$ | Wasserstoff oder $C_1$-$C_4$-Alkylgruppen; |
| $R^4$ | eine $C_5$-$C_{18}$-Alkylgruppe, die durch Sauerstoff unterbrochen sein kann, eine $C_5$-$C_{18}$-Alkenylgruppe, eine $C_4$-$C_{18}$-Alkinylgruppe oder eine Phenyl-$C_1$-$C_6$-alkylgruppe, wobei diese Gruppen bis zu drei der folgenden Substituenten tragen können: Hydroxyl, Halogen, Cyan, $C_1$-$C_7$-Alkoxy oder bis zu zwei Amino-, $C_1$-$C_4$-Alkylamino- oder Di-($C_1$-$C_4$)-alkylamino-Substituenten, und wobei der Phenylteil der Phenylalkylgruppe zusätzlich eine Phenoxygruppe oder bis zu 3 $C_2$-$C_4$-Alkenylgruppen, $C_1$-$C_4$-Alkoxy-$C_1$-$C_4$-alkylgruppen oder $C_1$-$C_6$-Alkylgruppen, die unsubstituiert oder partiell oder vollständig halogeniert sein können, tragen kann; oder eine $C_5$-$C_6$-Cycloalkyl-$C_1$-$C_8$-alkylgruppe, wobei der Cycloalkylring bis zu drei $C_1$-$C_4$-Alkylgruppen oder bis zu zwei Hydroxyl- oder Trifluormethylgruppen tragen kann; und |
| | eine $C_3$-$C_4$-Alkylgruppe, die durch Sauerstoff unterbrochen sein kann oder eine $C_4$-Alkenylgruppe, wobei diese Gruppen bis zu 3 der folgenden Substituenten tragen können: Hydroxyl, Halogen, Cyan, $C_1$-$C_7$-Alkoxy oder bis zu zwei Amino-, $C_1$-$C_4$-Alkylamino- oder $C_2$-$C_8$-Dialkylaminogruppen; |

oder gemeinsam mit dem Rest $R^3$ und dem Stickstoffatom einen 5- oder 6-gliedrigen heterocyclischen Ring, der mit $C_1$-$C_6$-Alkyl, Phenyl, $C_1$-$C_6$-Alkylphenyl ein- bis dreifach substituiert und durch ein Sauerstoffatom unterbrochen sein kann;

sowie der pflanzenverträglichen mineralsauren Salze I•HX und Metallkomplexe von I, dadurch gekennzeichnet, daß man entweder

a) ein Thiuronium-Salz IIa

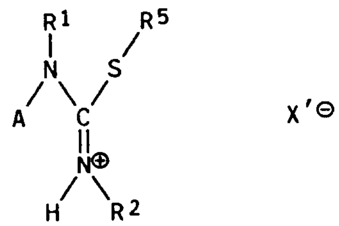

IIa

mit einem Amin IIIa

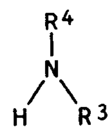

IIIa

oder ein Thiuronium-Salz IIb

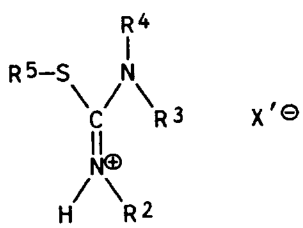

IIb

mit einem Amin IIIb

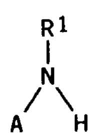

IIIb

oder ein Thiuronium-Salz IIc

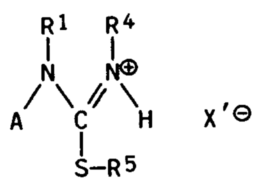

IIc

mit einem Amin IIIc

$R^2$-$NH_2$ IIIc

wobei $R^5$ eine Benzyl- oder $C_1$-$C_4$-Alkylgruppe bedeutet und x' für Halogen, Sulfat, Methylsulfat, $C_1$-$C_4$-Alkylsulfonat oder p-Toluylsulfonat steht, gewünschtenfalls in Gegenwart einer Hilfsbase umsetzt

oder
b) eine Aminoiminomethansulfonsäure IVa

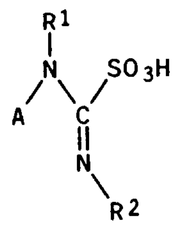

IVa

mit einem Amin IIIa,
oder eine Aminoiminomethansulfonsäure IVb

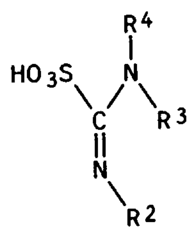

IVb

mit einem Amin IIIb,
oder eine Aminoiminomethansulfonsäure IVc oder IVd

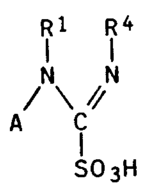 (IVc) 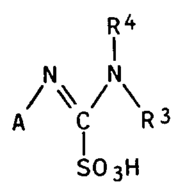 IVd

mit einem Amin IIIc umsetzt,
oder
c) ein Carbodiimid Va

$A\text{-}N = C = N\text{-}R^2$ Va

mit einem Amin IIIa,

oder ein Carbodiimid Vb

$R^2\text{-}N = C = N\text{-}R^4$ Vb

mit einem Amin IIIb,

oder ein Carbodiimid Vc

$A\text{-}N = C = N\text{-}R^4$ Vc

mit einem Amin IIIc umsetzt,
oder

d) ein Diphenylimidocarbonat VIa

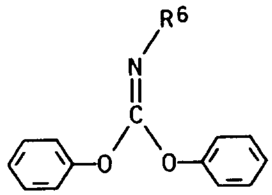

VIa

wobei $R^6$ eine Cyan-, Benzoyl- oder Methansulfonylgruppe bedeutet, stufenweise mit den beiden Aminen IIIa und IIIb umsetzt und anschließend hydrolysiert, oder

e) aus Bromcyan mit dem Amin IIIa das N-substiutierte Cyanamid VIIa

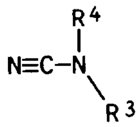

VIIa

herstellt und dieses anschließend mit dem mineralsauren Salz des Amins IIIb zu dem entsprechenden Salz der Verbindung I umsetzt,

oder mit Bromcyan aus dem Amin IIIb das N-substituierte Cyanamid VIIb

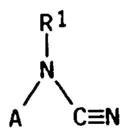

VIIb

herstellt und dieses anschließend mit dem mineralsauren Salz des Amins IIIa zu dem entsprechenden Salz der Verbindung I umsetzt,

und die erhaltenen Umsetzungsprodukte, soweit sie nicht bereits in Form der freien Basen oder der pflanzenverträglichen Salze anfallen, in die freien Basen bzw. die pflanzenverträglichen Salze überführt oder aus den freien Basen die Metallkomplexe herstellt.

**2.** Verwendung der Guanidine I, deren pflanzenverträglichen Salzen und Metallkomplexen gemäß Anspruch 1 als Fungizide.

**3.** Fungizides Mittel, enthaltend mindestens ein Guanidin der Formel I, dessen pflanzenverträgliches Salz oder dessen Metallkomplex gemäß Anspruch 1 und einen flüssigen oder festen Trägerstoff.

**4.** Verfahren zur Bekämpfung von Pilzen, dadurch gekennzeichnet, daß man eine fungizide wirksame Menge eines Guanidins der Formel I, dessen Salz oder Metallkomplex, gemäß Anspruch 1, auf Pilze, vom Pilzbefall bedrohte Pflanzen, deren Lebensraum oder auf das Saatgut der bedrohten Pflanzen einwirken läßt.